# EUROPEAN PATENT APPLICATION

(11) **EP 4 477 649 A1**
(43) Date of publication of application: **18.12.2024**
(21) Application number: 23749358.0
(22) Date of filing: 06.02.2023
(51) Int. Cl.: C07D 237/26, C07C 55/14

(54) **PREPARATION METHOD OF PYRIDAZINONE DERIVATIVE, AND INTERMEDIATE THEREOF**

(30) Priority: 07.02.2022 CN 202210116976
(71) Applicant: Xizang Haisco Pharmaceutical Co., Ltd., Lhoka, Tibet 856099 (CN)
(72) Inventor: FAN, Jiang, Chengdu, Sichuan 611130 (CN); DOU, Ying, Chengdu, Sichuan 611130 (CN); ZHU, Fengfei, Chengdu, Sichuan 611130 (CN); LIU, Zhenping, Chengdu, Sichuan 611130 (CN); FENG, Jianchuan, Chengdu, Sichuan 611130 (CN); WANG, Zhigang, Chengdu, Sichuan 611130 (CN); SUN, Jingxiong, Chengdu, Sichuan 611130 (CN); LIU, Sijia, Chengdu, Sichuan 611130 (CN)
(74) Representative: Rückerl, Florian
(86) International application number: PCT/CN2023/074589
(87) International publication number: WO 2023/147779

(57) **Abstract**

Provided are a preparation method of a pyridazinone derivative, an intermediate thereof, and a preparation method of the intermediate. The method has the advantages of easily available raw materials, simple steps, low costs, good intermediate stability, high purity and high yield, and is suitable for large-scale industrial production.

## Description

### Technical Field

The present invention relates to the field of medicine, and in particular, the present invention relates to a preparation method of a pyridazinone derivative, an intermediate thereof, and a preparation method of the intermediate.

### Background Art

Classical familial hypercholesterolemia (FH) is an autosomal (co) dominant genetic disorder, with the main clinical manifestation of a marked increase in serum low density lipoprotein-cholesterol (LDL-C) level, as well as skin/tendon xanthoma, and can lead to early cardiovascular disease. Common FHs are divided into heterozygous familial hypercholesterolemia (HeFH) and, more rarely, homozygous familial hypercholesterolemia (HoFH). According to statistics, the prevalence of HeFH is as high as 0.2%-0.48%. FH is usually caused by inactivating mutations in the LDL receptor (LDLR). In clinical use, statin medications are the drugs of first choice for treatment, not only to reduce LDL-C levels, but also to improve the prognosis of the patients with FH. Patients who do not respond well to statin therapy or have adverse reactions can be treated in combination with the cholesterol absorption inhibitor of ezetimibe. PCSK9 inhibitor may be additionally administered to patients who do not reach the standard cholesterol level after the treatments described above. Although there are corresponding treatments with respect to HeFH, many patients (up to 40% of HeFH patients) are unable to reach the standard level of cholesterol (LDL-C) after these treatments, and the accumulation of cholesterol in their bodies becomes a lifelong burden.

Thyroid hormone receptors are divided into two subtypes, α and β subtypes. Binding of thyroid hormone to the β subtype receptor can promote the cholesterol metabolism. As a result, several thyroid hormone analogs or thyromimetics that selectively agonize the β subtype receptor have been developed in recent years. The oral agonist of MGL-3196, developed by the Madrigal company, has been shown to significantly reduce the LDL-C levels in patients, and ameliorate the metabolic syndromes (such as insulin resistance and dyslipidemia) and fatty liver diseases (including lipid toxicity and inflammation).

In the compound patent WO 2019240938A1, a compound of formula (I), which is a thyroxine beta receptor agonist and is intended for the treatment of primary hypercholesterolemia of adult, is described and a preparation method therefor is also disclosed.

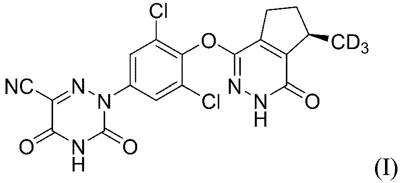

However, this preparation method has the disadvantages of poor intermediate stability, difficult purification, requiring multiple steps of column purification which is not conducive to industrial application, reaction conditions that are not conducive to large-scale production, low yield, and high cost.

### Summary of the Invention

An object of the present invention is to provide a preparation method of a pyridazinone derivative.

The method has the advantages of easily available raw materials, simple steps, that is, the product is purified by crystallization instead of column chromatography on silica gel throughout the entire synthesis process, low costs, good intermediate stability, high purity and high yield, and is suitable for large-scale industrial production.

Another object of the present invention is to provide an intermediate for the preparation of the pyridazinone derivative.

Another object of the present invention is to provide a preparation method of the intermediate for the preparation of the pyridazinone derivative.

The present invention provides compounds of formulae (1E) and (1H) or a pharmaceutically acceptable salt thereof,

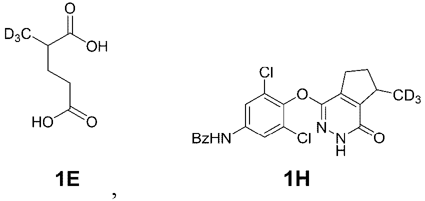

The present invention provides a preparation method of a compound of formula (1F), comprising the steps of 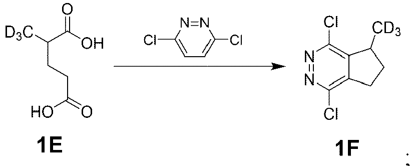
after the reaction of a compound 1E with 3,6-dichloropyridazine, water and silver nitrate, adding an acid A and an oxidant for reaction to obtain the compound 1F.
adding the acid A and the oxidant to a mixture of the compound 1E, 3,6-dichloropyridazine, water and silver nitrate, and reacting same at 55-65°C to obtain the compound 1F.

In some embodiments of the present invention, adding water to the compound 1E and 3,6-dichloropyridazine, raising the temperature to 30-40°C, adding silver nitrate and raising the temperature to 55-65°C, adding the acid A and the oxidant, and reacting same at 70-80°C to obtain the compound 1F.

In some embodiments of the present invention, the acid A is selected from trifluoroacetic acid, pentafluoroacetic acid, heptafluoropropionic acid, sulfuric acid, hydrochloric acid, phosphoric acid, nitric acid, formic acid, acetic acid, benzenesulfonic acid, methanesulfonic acid and p-toluenesulfonic acid; in some embodiments of the present invention, the acid A is selected from trifluoroacetic acid; in some embodiments of the present invention, the oxidant is selected from ammonium persulfate, potassium persulfate, sodium persulfate, t-butylhydroperoxide and hydrogen peroxide; in some embodiments of the present invention, the oxidant is selected from ammonium persulfate; in some embodiments of the present invention, the concentration of ammonium persulfate is 0.1-1 g/mL.

In some embodiments of the present invention, the molar ratio of the compound 1E to 3,6-dichloropyridazine is 1 : 0.9-5.0; In some embodiments of the present invention, the molar ratio of the compound 1E to 3,6-dichloropyridazine is 1 : 1.0-2.0; in some embodiments of the present invention, the molar ratio of the compound 1E to silver nitrate is 1 : 0.2-2.0; in some embodiments of the present invention, the molar ratio of the compound 1E to silver nitrate is 1 : 0.4-1.0; in some embodiments of the present invention, the molar ratio of the compound 1E to the acid A is 1 : 0.05-2.0; and in some embodiments of the present invention, the molar ratio of the compound 1E to the acid A is 1 : 0.1-1.0.

In the compound patent (WO 2019240938A1), an intermediate material 3,6-dichloro-1,2,4,5-tetrazine is used to prepare 1F. Compared with the intermediate material 3,6-dichloropyridazine of the present invention, the intermediate material 3,6-dichloro-1,2,4,5-tetrazine has high cost, poor stability, low yield of a final product, and is not suitable for industrial production.

The present invention provides a preparation method of a compound of formula (1E), comprising the steps of 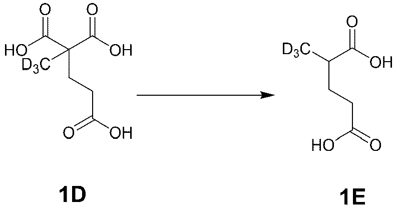 reacting a compound 1D with stirring at 100-180°C to obtain the compound 1E.

In some embodiments of the present invention, a compound 1D is reacted with stirring at 120-150°C to obtain the compound 1E, and after the reaction with stirring, the post-treatment and drying steps are further involved, the post-treatment comprises the addition of organic solvents F and A at 20-50°C, crystallization at room temperature and filtration; in some embodiments of the present invention, the compound 1D is reacted with stirring at 130-140°C to obtain the compound 1E; in some embodiments of the present invention, the post-treatment comprises the addition of organic solvents F and A at 30-40°C, crystallization at room temperature, filtration, and collection of filter cake.

In some embodiments of the present invention, the organic solvent F is selected from dichloromethane, methyl acetate, acetonitrile, toluene, ethyl acetate, isopropyl acetate, acetone, 2-methyltetrahydrofuran, tetrahydrofuran, 1,4-dioxane, dimethyl carbonate, propylene glycol methyl ether acetate and dimethyl nylon acid; in some embodiments of the present invention, the organic solvent F is selected from dichloromethane, methyl acetate, dimethyl carbonate, propylene glycol methyl ether acetate and dimethyl nylon acid; in some embodiments of the present invention, the organic solvent F is selected from dichloromethane; in some embodiments of the present invention, the organic solvent A is selected from alkyl hydrocarbons or esters; in some embodiments of the present invention, the organic solvent A is selected from n-pentane, n-hexane, n-heptane, petroleum ether, 2-methylpentane, 3-methylpentane, 2,3-dimethylbutane, 2,2-dimethylbutane, methyl acetate, dimethyl carbonate, propylene glycol methyl ether acetate, dimethyl nylon acid, ethyl acetate and isopropyl acetate; in some embodiments of the present invention, the organic solvent A is selected from n-pentane, n-hexane, n-heptane, petroleum ether, 2-methylpentane, 3-methylpentane, 2,3-dimethylbutane and 2,2-dimethylbutane; in some embodiments of the present invention, the organic solvent A is selected from n-hexane; in some embodiments of the present invention, the drying comprises drying the filter cake after the filtration treatment at 40-60°C and a vacuum degree of ≤ -0.07 MPa; and in some embodiments of the present invention, the drying comprises drying the filter cake after the filtration treatment at 45-55°C and a vacuum degree of ≤ -0.07 MPa.

In some embodiments of the present invention, a preparation method of a compound of formula (1E) further comprises the steps of 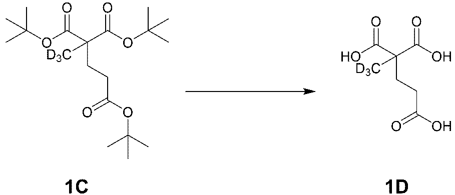
reacting a compound 1C with an organic solvent F and an acid A under reflux to obtain the compound 1D;
reacting the compound 1C with the organic solvent F and the acid A under reflux, followed by concentration under reduced pressure, pulping, filtration and drying to obtain the compound 1D.

In some embodiments of the present invention, the organic solvent F is selected from dichloromethane, methyl acetate, acetonitrile, toluene, ethyl acetate, isopropyl acetate, acetone, 2-methyltetrahydrofuran, tetrahydrofuran, 1,4-dioxane, dimethyl carbonate, propylene glycol methyl ether acetate and dimethyl nylon acid; in some embodiments of the present invention, the organic solvent F is selected from dichloromethane, methyl acetate, dimethyl carbonate, propylene glycol methyl ether acetate and dimethyl nylon acid; in some embodiments of the present invention, the organic solvent F is selected from dichloromethane; in some embodiments of the present invention, the acid A is selected from trifluoroacetic acid, pentafluoroacetic acid, heptafluoropropionic acid, hydrochloric acid, sulfuric acid, phosphoric acid, formic acid, benzenesulfonic acid, methanesulfonic acid and p-toluenesulfonic acid; in some embodiments of the present invention, the acid A is selected from trifluoroacetic acid, pentafluoroacetic acid and heptafluoropropionic acid; in some embodiments of the present invention, the acid A is selected from trifluoroacetic acid; in some embodiments of the present invention, the organic solvent A is selected from alkyl hydrocarbons or esters; in some embodiments of the present invention, the organic solvent A is selected from n-pentane, n-hexane, n-heptane, petroleum ether, 2-methylpentane, 3-methylpentane, 2,3-dimethylbutane, 2,2-dimethylbutane, methyl acetate, dimethyl carbonate, propylene glycol methyl ether acetate, dimethyl nylon acid, ethyl acetate and isopropyl acetate; in some embodiments of the present invention, the organic solvent A is selected from n-pentane, n-hexane, n-heptane, petroleum ether, 2-methylpentane, 3-methylpentane, 2,3-dimethylbutane and 2,2-dimethylbutane; in some embodiments of the present invention, the organic solvent A is selected from n-hexane; in some embodiments of the present invention, the drying comprises drying the filter cake after the filtration treatment at 40-60°C and a vacuum degree of ≤ -0.07 MPa; and in some embodiments of the present invention, the drying comprises drying the filter cake after the filtration treatment at 45-55°C and a vacuum degree of ≤ -0.07 MPa.
in some embodiments of the present invention, the molar ratio of the compound 1C to the acid A is 1 : 3.0-30.0; and in some embodiments of the present invention, the molar ratio of the compound 1C to the acid A is 1 : 3.0-10.0.

In some embodiments of the present invention, a preparation method of a compound of formula (1E) further comprises the steps of 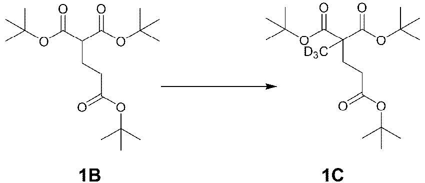 reacting a compound 1B with a deuterated reagent in the presence of an organic base to obtain the compound 1C.

In some embodiments of the present invention, the deuterated reagent is selected from deuterated iodomethane, deuterated bromomethane, deuterated chloromethane, D3-p-toluenesulfonyl methyl ester and D3-methylsulfonyl methyl ester; in some embodiments of the present invention, the deuterated reagent is selected from deuterated iodomethane.

In some embodiments of the present invention, the molar ratio of the compound 1B to the base is 1 : 1.00-5.05; in some embodiments of the present invention, the molar ratio of the compound 1B to the base is 1 : 1.05-2.05; in some embodiments of the present invention, the molar ratio of the compound 1B to the deuterated reagent is 1 : 0.9-5.0; and in some embodiments of the present invention, the molar ratio of the compound 1B to the deuterated reagent is 1 : 1.0- 2.5.

In some embodiments of the present invention, the preparation method further comprises the steps of 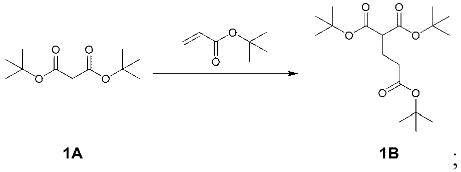 adding a compound 1A, a base and a catalyst to an organic solvent E, followed by adding t-butyl acrylate under the protection of an inert gas to obtain the compound 1B after the reaction is completed.

In some embodiments of the present invention, the base is selected from sodium methoxide, potassium ethoxide, potassium tert-butoxide, sodium tert-butoxide, lithium tert-butoxide, sodium acetate, potassium acetate, K₃PO₄, K₂CO₃, KHCO₃, Cs₂CO₃, Na₂CO₃, NaHCO₃, KF, pyridine, triethylamine, N,N-diisopropylethylamine and DBU; in some embodiments of the present invention, the base is selected from sodium methoxide, potassium ethoxide, potassium tert-butoxide and sodium tert-butoxide; in some embodiments of the present invention, the base is selected from potassium tert-butoxide; in some embodiments of the present invention,the catalyst is tetrabutylammonium bisulfate; in some embodiments of the present invention, the organic solvent E is selected from tetrahydrofuran, N,N-dimethylacetamide, dimethylformamide, acetonitrile, acetone, methyl isobutyl ketone, toluene, pyridine, dichloromethane, ethyl acetate, 2-methyltetrahydrofuran or 1,4-dioxane; in some embodiments of the present invention, the organic solvent E is selected from tetrahydrofuran, N,N-dimethylacetamide, dimethylformamide, acetonitrile and acetone; in some embodiments of the present invention, the organic solvent E is selected from tetrahydrofuran.

In some embodiments of the present invention, the molar ratio of the compound 1A to the base is 1 : 0.001-0.5; in some embodiments of the present invention, the molar ratio of the compound 1A to the base is 1 : 0.01-0.25; in some embodiments of the present invention, the molar ratio of the compound 1A to t-butyl acrylate is 1 : 0.8-6.0; and in some embodiments of the present invention, the molar ratio of the compound 1A to t-butyl acrylate is 1 : 1.0-3.0.

The present invention further provides a preparation method of a compound of formula (I), comprising the steps of 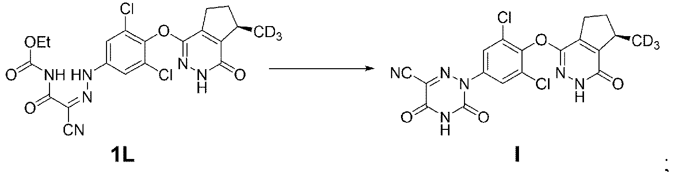 reacting a compound 1L in the presence of an organic solvent B and an alkali solution B to obtain the compound I.

In some embodiments of the present invention, the organic solvent B is selected from dimethylsulfoxide, acetonitrile, N,N-dimethylformamide, N,N-dimethylacetamide, hexamethylphosphoramide and N-methylpyrrolidone; and in some embodiments of the present invention, the organic solvent B is selected from N,N-dimethylacetamide.

In some embodiments of the present invention, the compound 1L is reacted in the presence of the organic solvent B and the alkali solution B at 100-130°C to obtain the compound I; in some embodiments of the present invention, the compound 1L is reacted in the presence of the organic solvent B and the alkali solution B at 110-120°C to obtain the compound I; in some embodiments of the present invention, the compound 1L is reacted in the presence of the organic solvent B and the alkali solution B at 115°C to obtain the compound I.

In some embodiments of the present invention, the molar ratio of the compound 1L to the alkali solution B is 1 : 0.5-1 : 5.0; in some embodiments of the present invention, the molar ratio of the compound 1L to the alkali solution B is 1 : 0.5-1 : 4.5; in some embodiments of the present invention, the molar ratio of the compound 1L to the alkali solution B is 1 : 1-1 : 4.5; in some embodiments of the present invention, the molar ratio of the compound 1L to the alkali solution B is 1 : 2-1 : 4.5; in some embodiments of the present invention, the molar ratio of the compound 1L to the alkali solution B is 1 : 1-3 : 4.5; in some embodiments of the present invention, the molar ratio of the compound 1L to the alkali solution B is 1 : 0.5-1 : 4.0; in some embodiments of the present invention, the molar ratio of the compound 1L to the alkali solution B is 1 : 0.5-1 : 3.5; in some embodiments of the present invention, the molar ratio of the compound 1L to the alkali solution B is 1 : 0.5-1 : 3.0; in some embodiments of the present invention, the molar ratio of the compound 1L to the alkali solution B is 1 : 1.0-2.5; and in some embodiments of the present invention, the molar ratio of the compound 1L to the alkali solution B is 1 : 1.0-2.0.

In some embodiments of the present invention, the compound 1L is reacted in the presence of the organic solvent B and the alkali solution B, and after the reaction, the post-treatment and refining steps are further involved to obtain the compound I; in some embodiments of the present invention, the post-treatment comprises the addition of water at 55-65°C, filtration, first washing, followed by the addition of an organic solvent D and water, crystallization at 5-15°C, filtration, second washing and drying to obtain a crude of the compound I; in some embodiments of the present invention, the refining comprises mixing the crude of the compound I with anhydrous ethanol, stirring same for reaction at 70-80°C for 3-6 h, and cooling to 0-10°C for crystallization, after the crystallization is completed, filtering, washing and drying same to obtain the compound I; in some embodiments of the present invention, the first washing is performed with N,N-dimethylacetamide; the organic solvent D is formic acid, acetic acid, oxalic acid and propionic acid; in some embodiments of the present invention, the organic solvent D is acetic acid; and in some embodiments of the present invention, the second washing is performed with water and anhydrous ethanol, respectively.

A compound I synthesized in the compound patent (WO 2019240938A1) is purified by preparative HPLC; combined with the previous step, the two-step yield is only 20-30%, which is low; while in the process of the present invention, this two-step yield is as high as 70-80%.

In some embodiments of the present invention, a preparation method of a compound of formula (I) further comprises the steps of 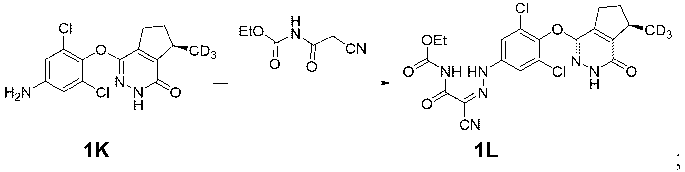 dissolving a compound 1K in water and an organic solvent D, adding an acid B and stirring same, then adding an alkali solution A and an alkali solution B successively, and finally adding N-cyanoacetylurethane for reaction to obtain the compound 1L.

In some embodiments of the present invention, the compound 1K is dissolved in water and the organic solvent D. At -5-5°C, the acid B is added and stirred for reaction, then the alkali solution A is added, after the reaction is completed, the alkali solution B is added, N-cyanoacetylurethane is finally added, and the temperature is raised to 5-15°C for reaction to obtain the compound 1L.

In some embodiments of the present invention, the organic solvent D is selected from formic acid, acetic acid, oxalic acid and propionic acid; in some embodiments of the present invention, the organic solvent D is selected from acetic acid; in some embodiments of the present invention, the acid B is selected from hydrochloric acid, nitric acid, sulfuric acid, phosphoric acid and perchloric acid; in some embodiments of the present invention, the acid B is selected from hydrochloric acid; in some embodiments of the present invention, the alkali solution A is a nitrite selected from sodium nitrite, potassium nitrite and ammonium nitrite; in some embodiments of the present invention, the nitrite is selected from sodium nitrite; in some embodiments of the present invention, the concentration of sodium nitrite is 0.3-0.6 g/mL; in some embodiments of the present invention, the alkali solution B is an acetate selected from sodium acetate, potassium acetate and ammonium acetate; in some embodiments of the present invention, the acetate salt is selected from sodium acetate; and in some embodiments of the present invention, the concentration of the sodium acetate is 0.1-0.3 g/mL.

In some embodiments of the present invention, the molar ratio of the compound 1K to N-cyanoacetylurethane is 1 : 0.9-5.0; in some embodiments of the present invention, the molar ratio of the compound 1K to N-cyanoacetylurethane is 1 : 1.0-3.0; in some embodiments of the present invention, the molar ratio of the compound 1K to the base A is 1 : 1.0-10.0; and in some embodiments of the present invention, the molar ratio of the compound 1K to the base A is 1 : 1.1-3.0.

The base added in the compound patent (WO 2019240938A1) is pyridine and the post-treatment comprises extraction with ethyl acetate and concentration to obtain a product I. In the process of the present invention, water is added to directly crystallize after the reaction is completed, the post-treatment is simple and easy to scale up, and the product has a higher yield and purity.

In some embodiments of the present invention, a preparation method of a compound of formula (I) further comprises the steps of 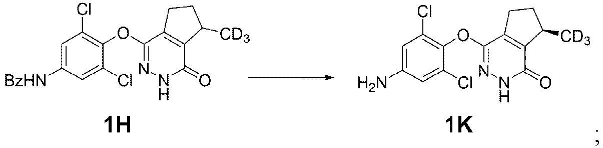 mixing a compound 1H with an alkali solution C and an organic solvent C, and reacting same under reflux to obtain the compound 1K.

In some embodiments of the present invention, the alkali solution C is selected from a potassium hydroxide solution, a sodium hydroxide solution, a lithium hydroxide solution, a potassium tert-butoxide solution, a lithium tert-butoxide solution, a sodium carbonate solution, a potassium carbonate solution and a cesium carbonate solution; in some embodiments of the present invention, the alkali solution C is selected from potassium hydroxide; in some embodiments of the present invention, the alkali solution C is selected from sodium hydroxide; in some embodiments of the present invention, the mass concentration of the potassium hydroxide is 0.1-0.2 g/mL; in some embodiments of the present invention, the organic solvent C is selected from anhydrous methanol, anhydrous ethanol, n-propanol, isopropanol, tert-butanol, n-butanol, toluene, acetone, methyl isobutyl ketone, tetrahydrofuran, 2-methyltetrahydrofuran, dimethylsulfoxide, N,N-dimethylacetamide, hexamethylphosphoramide and N-methylpyrrolidone; in some embodiments of the present invention, the organic solvent C is selected from anhydrous ethanol; and in some embodiments of the present invention, the organic solvent C is selected from anhydrous methanol.

In some embodiments of the present invention, the reflux reaction temperature is 70-90°C; in some embodiments of the present invention, the reflux reaction temperature is 75-85°C; in some embodiments of the present invention, the reflux reaction temperature is 80 ± 5°C; and in some embodiments of the present invention, the reflux reaction temperature is 80°C.

In some embodiments of the present invention, the compound 1H is mixed with the alkali solution C and the organic solvent C and heated under reflux for reaction for 15-20 h to obtain a racemate of the compound 1K; and the racemate of the compound 1K is then post-treated and refined, and finally resolved by chiral SFC to obtain the compound 1K.

In some embodiments of the present invention, the post-treatment comprises cooling to 20-30°C, adding water, adjusting the pH of the solution to 8-9, extraction, washing, drying and filtration; in some embodiments of the present invention, the refining comprises completely dissolving the crude obtained after post-treatment with a dioxane solution, crystallizing same at room temperature, and filtering same; and the refining is repeated 2-3 times and drying is performed under vacuum.

In some embodiments of the present invention, a preparation method of a compound of formula (I) further comprises the steps of 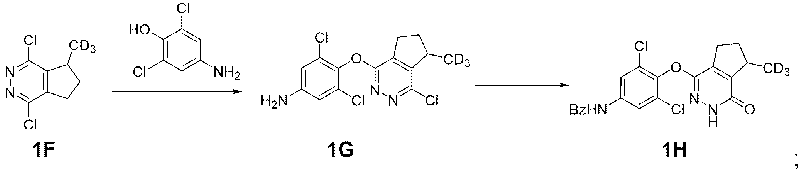
reacting a compound 1F and 4-amino-2,6-dichlorophenol in the presence of an organic solvent B and cesium carbonate under the protection of an inert gas to obtain a compound 1G; and
reacting the compound 1G with benzoic anhydride in the presence of glacial acetic acid to obtain the compound 1H.

In the compound patent (WO 2019240938A1), 1G is prepared by using cuprous iodide as a catalyst, the yield is low, and there may be residual metallic copper, which requires column purification; while in the present process, 1G is obtained by direct substitution reaction under alkaline conditions, and a crude is continuously added in three steps to obtain a racemate of 1k, which is then purified by crystallization, and the three-step telescopic reaction is more suitable for industrial production; in addition, in the compound patent, when the racemate compound of 1K is prepared, its yield in three steps is only 20-25%; whereas in the process of the present invention, when the racemate compound of 1K is prepared, its yield in three steps can up to 30-40%; and the compound 1H has different protecting groups, which makes it easier to crystallize and greatly simplifies the post-treatment process.

In some embodiments of the present invention, the preparation method specifically comprises the steps of
(a) mixing a compound 1F, 4-amino-2,6-dichlorophenol and an organic solvent B, adding cesium carbonate, and heating same to 90-95°C under the protection of nitrogen for reaction for 18-22 h to obtain a compound 1G; and
(b) dissolving the compound 1G obtained in step (a) in glacial acetic acid completely and adding benzoic anhydride, drying same after the reaction and raising the temperature to 115-125°C for reaction for 15-20 h, so as to obtain the compound 1H after post-treatment.

In some embodiments of the present invention, the organic solvent B is selected from dimethylsulfoxide, acetonitrile, N,N-dimethylformamide, N,N-dimethylacetamide, hexamethylphosphoramide and N-methylpyrrolidone; and in some embodiments of the present invention, the organic solvent B is selected from N,N-dimethylacetamide.

In some embodiments of the present invention, the post-treatment comprises concentrating the reaction solution under reduced pressure at 60-65°C, adding anhydrous ethanol, slowly adding the mixed solution into water, quickly stirring to precipitate solid, centrifuging and washing same to obtain a compound 1H.

In some embodiments of the present invention, the molar ratio of the compound 1F, 4-amino-2,6-dichlorophenol and cesium carbonate is 1-5 : 1-5 : 1.0-10; in some embodiments of the present invention, the molar ratio of the compound 1F, 4-amino-2,6-dichlorophenol and cesium carbonate is 2-3 : 2-3 : 3.0-5.0; in some embodiments of the present invention, the molar ratio of the benzoic anhydride to the compound 1F is 1-5 : 1-5; in some embodiments of the present invention, the molar ratio of the benzoic anhydride to the compound 1F is 2-3 : 2.2-3.5;
in some embodiments of the present invention, a preparation method of a compound of formula (I) further comprises the steps of 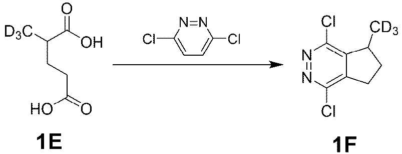 ,
after the reaction of a compound 1E with 3,6-dichloropyridazine, water and silver nitrate, adding an acid A and an oxidant for reaction to obtain the compound 1F.
adding water and silver nitrate to a mixture of the compound 1E and 3,6-dichloropyridazine, then adding the acid A and the oxidant thereto and reacting same at 55-65°C to obtain the compound 1F.

In some embodiments of the present invention, adding water to the compound 1E and 3,6-dichloropyridazine, raising the temperature to 30-40°C, adding silver nitrate and raising the temperature to 55-65°C, adding the acid A and the oxidant, and reacting same at 70-80°C to obtain the compound 1F.

In some embodiments of the present invention, the acid A is selected from trifluoroacetic acid, pentafluoroacetic acid, heptafluoropropionic acid, sulfuric acid, hydrochloric acid, phosphoric acid, nitric acid, formic acid, acetic acid, benzenesulfonic acid, methanesulfonic acid and p-toluenesulfonic acid; in some embodiments of the present invention, the acid A is selected from trifluoroacetic acid; in some embodiments of the present invention, the oxidant is selected from ammonium persulfate, potassium persulfate, sodium persulfate, t-butylhydroperoxide and hydrogen peroxide; in some embodiments of the present invention, the oxidant is selected from ammonium persulfate; in some embodiments of the present invention, the concentration of ammonium persulfate is 0.1-1 g/mL.

In some embodiments of the present invention, the molar ratio of the compound 1E to 3,6-dichloropyridazine is 1 : 0.9-5.0; in some embodiments of the present invention, the molar ratio of the compound 1E to 3,6-dichloropyridazine is 1 : 1.0-2.0; in some embodiments of the present invention, the molar ratio of the compound 1E to silver nitrate is 1 : 0.2-2.0; in some embodiments of the present invention, the molar ratio of the compound 1E to silver nitrate is 1 : 0.4-1.0; in some embodiments of the present invention, the molar ratio of the compound 1E to the acid A is 1 : 0.05-2.0; and in some embodiments of the present invention, the molar ratio of the compound 1E to the acid A is 1 : 0.1-1.0.

A first solution of the present invention provides compounds of formulae (1E) and (1H), or a pharmaceutically acceptable salt thereof,

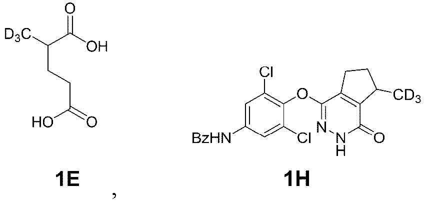

A second solution of the present invention provides a preparation method of a compound of formula (1F), comprising the steps of 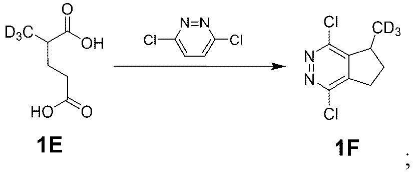
after the reaction of a compound 1E with 3,6-dichloropyridazine, water and silver nitrate, adding an acid A and an oxidant for reaction to obtain the compound 1F.
adding the acid A and the oxidant to a mixture of the compound 1E, 3,6-dichloropyridazine, water and silver nitrate, and reacting same at 55-65°C to obtain the compound 1F;
wherein the acid A is selected from trifluoroacetic acid, pentafluoroacetic acid, heptafluoropropionic acid, sulfuric acid, hydrochloric acid, phosphoric acid, nitric acid, formic acid, acetic acid, benzenesulfonic acid, methanesulfonic acid and p-toluenesulfonic acid; and the oxidant is selected from ammonium persulfate, potassium persulfate, sodium persulfate, t-butylhydroperoxide and hydrogen peroxide.

A third solution of the present invention provides a preparation method of a compound of formula (1E), comprising the steps of 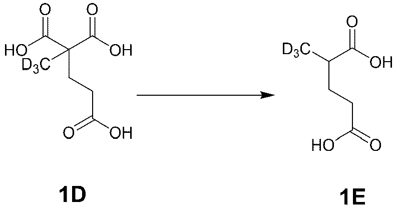
reacting a compound 1D with stirring at 100-180°C to obtain the compound 1E;
after the reaction with stirring, the post-treatment and drying steps are further involved, the post-treatment comprises the addition of organic solvents F and A at 20-50°C, crystallization at room temperature and filtration;
further comprising the steps of 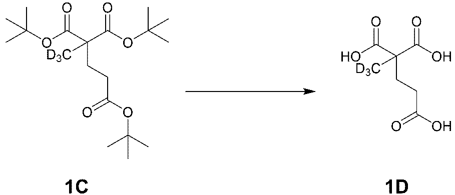
reacting the compound 1C with the organic solvent F and the acid A under reflux, followed by concentration under reduced pressure, pulping, filtration and drying to obtain the compound 1D;
wherein the organic solvent F is selected from dichloromethane, methyl acetate, dimethyl carbonate, propylene glycol methyl ether acetate, dimethyl nylon acid, acetonitrile, toluene, ethyl acetate, isopropyl acetate, acetone, 2-methyltetrahydrofuran, tetrahydrofuran and 1,4-dioxane; and the acid A is selected from trifluoroacetic acid, pentafluoroacetic acid, heptafluoropropionic acid, hydrochloric acid, sulfuric acid, phosphoric acid, formic acid, benzenesulfonic acid, methanesulfonic acid and p-toluenesulfonic acid; the organic solvent A is selected from n-pentane, n-hexane, n-heptane, petroleum ether, 2-methylpentane, 3-methylpentane, 2,3-dimethylbutane, 2,2-dimethylbutane, methyl acetate, dimethyl carbonate, propylene glycol methyl ether acetate, dimethyl nylon acid, ethyl acetate and isopropyl acetate; and the drying comprises drying the filter cake after the filtration treatment at 40-60°C and a vacuum degree of ≤ -0.07 MPa;
further comprising the steps of 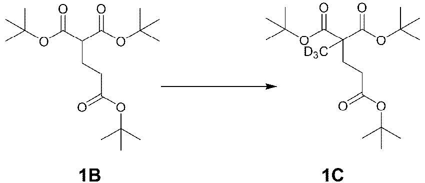
reacting a compound 1B with a deuterated reagent in the presence of a base to obtain the compound 1C;
wherein the deuterated reagent is selected from deuterated iodomethane, deuterated bromomethane, deuterated chloromethane, D₃-p-toluenesulfonyl methyl ester and D₃-methylsulfonyl methyl ester; preferably deuterated iodomethane;
further comprising the steps of 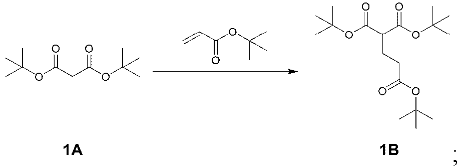
adding a compound 1A, a base and a catalyst to an organic solvent E, followed by adding t-butyl acrylate under the protection of an inert gas to obtain the compound 1B after the reaction is completed;
the base is selected from sodium methoxide, potassium ethoxide, potassium tert-butoxide, sodium tert-butoxide, lithium tert-butoxide, sodium acetate, potassium acetate, K₃PO₄, K₂CO₃, KHCO₃, Cs₂CO₃, Na₂CO₃, NaHCO₃, KF, pyridine, triethylamine, N,N-diisopropylethylamine and DBU; preferably potassium tert-butoxide; the catalyst is tetrabutylammonium bisulfate; and the organic solvent E is selected from tetrahydrofuran, N,N-dimethylacetamide, dimethylformamide, acetonitrile, acetone, methyl isobutyl ketone, toluene, pyridine, dichloromethane, ethyl acetate, 2-methyltetrahydrofuran or 1,4-dioxane.

A fourth solution of the present invention provides a preparation method of a compound of formula (I), comprising the steps of 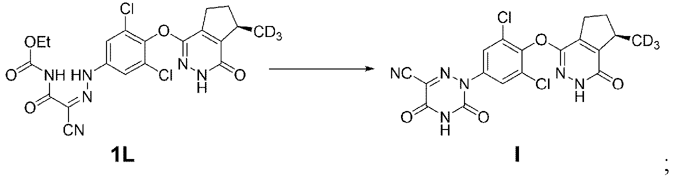
reacting a compound 1L in the presence of an organic solvent B and an alkali solution B to obtain the compound I;
wherein the organic solvent B is selected from dimethylsulfoxide, acetonitrile, N,N-dimethylformamide, N,N-dimethylacetamide, hexamethylphosphoramide and N-methylpyrrolidone; preferably N,N-dimethylacetamide;
further comprising the steps of 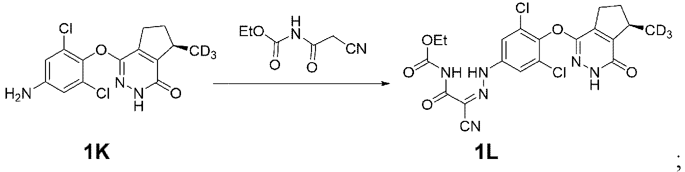
dissolving a compound 1K in water and an organic solvent D, adding an acid B and stirring same, then adding an alkali solution A and an alkali solution B successively, and finally adding N-cyanoacetylurethane for reaction to obtain the compound 1L;
wherein the organic solvent D is selected from formic acid, acetic acid, oxalic acid and propionic acid; preferably acetic acid; the acid B is selected from hydrochloric acid, nitric acid, sulfuric acid, phosphoric acid and perchloric acid; preferably hydrochloric acid; the alkali solution A is a nitrite selected from sodium nitrite, potassium nitrite and ammonium nitrite; and preferably sodium nitrite; and the alkali solution B is an acetate selected from sodium acetate, potassium acetate and ammonium acetate; preferably sodium acetate;
further comprising the steps of 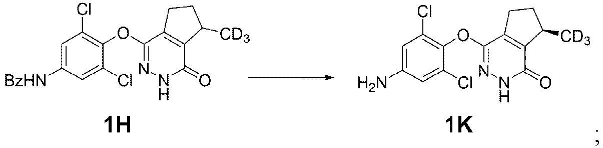
mixing a compound 1H with an alkali solution C and an organic solvent C, and reacting same under reflux to obtain the compound 1K;
wherein the alkali solution C is selected from a potassium hydroxide solution, a sodium hydroxide solution, a lithium hydroxide solution, a potassium tert-butoxide solution, a lithium tert-butoxide solution, a sodium carbonate solution, a potassium carbonate solution and a cesium carbonate solution; preferably potassium hydroxide and sodium hydroxide; and the organic solvent C is anhydrous methanol, anhydrous ethanol, n-propanol, isopropanol, tert-butanol, n-butanol, toluene, acetone, methyl isobutyl ketone, tetrahydrofuran, 2-methyltetrahydrofuran, dimethylsulfoxide, N,N-dimethylacetamide, hexamethylphosphoramide and N-methylpyrrolidone; preferably anhydrous ethanol and anhydrous methanol;
further comprising the steps of 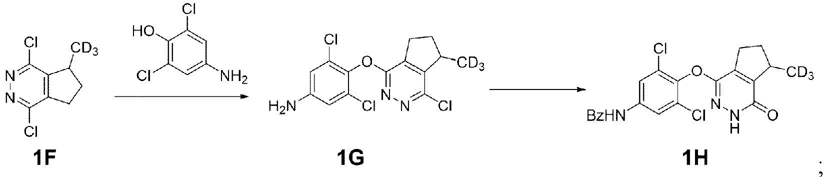
reacting a compound 1F and 4-amino-2,6-dichlorophenol in the presence of an organic solvent B and cesium carbonate under the protection of an inert gas to obtain a compound 1G; and
reacting the compound 1G with benzoic anhydride in the presence of glacial acetic acid to obtain the compound 1H;
wherein the organic solvent B is selected from dimethylsulfoxide, acetonitrile, N,N-dimethylformamide, N,N-dimethylacetamide, hexamethylphosphoramide and N-methylpyrrolidone; preferably N,N-dimethylacetamide;
further comprising the steps of 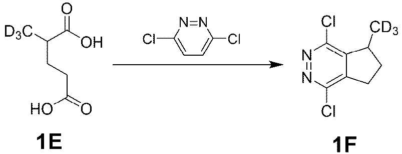
after the reaction of a compound 1E with 3,6-dichloropyridazine, water and silver nitrate, adding an acid A and an oxidant for reaction to obtain the compound 1F.
adding water and silver nitrate to a mixture of the compound 1E and 3,6-dichloropyridazine, then adding the acid A and the oxidant thereto and reacting same at 55-65°C to obtain the compound 1F;
wherein the acid A is selected from trifluoroacetic acid, pentafluoroacetic acid, heptafluoropropionic acid, sulfuric acid, hydrochloric acid, phosphoric acid, nitric acid, formic acid, acetic acid, benzenesulfonic acid, methanesulfonic acid and p-toluenesulfonic acid; and preferably trifluoroacetic acid; and the oxidant is selected from ammonium persulfate, potassium persulfate, sodium persulfate, t-butylhydroperoxide and hydrogen peroxide; preferably ammonium persulfate.

A fifth solution of the present invention provides a preparation method of a compound of formula (I), comprising the steps of
(1) adding water and silver nitrate to a mixture of the compound 1E and 3,6-dichloropyridazine and reacting same, then adding the acid A and the oxidant thereto and reacting same to obtain the compound 1F; 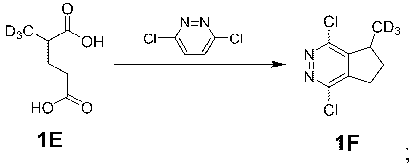 wherein the acid A is selected from trifluoroacetic acid, pentafluoroacetic acid, heptafluoropropionic acid, sulfuric acid, hydrochloric acid, phosphoric acid, nitric acid, formic acid, acetic acid, benzenesulfonic acid, methanesulfonic acid and p-toluenesulfonic acid; and preferably trifluoroacetic acid; and the oxidant is selected from ammonium persulfate, potassium persulfate, sodium persulfate, t-butylhydroperoxide and hydrogen peroxide; preferably ammonium persulfate;
(2) reacting the compound 1F obtained in step (1) and 4-amino-2,6-dichlorophenol in the presence of an organic solvent B and cesium carbonate under the protection of an inert gas to obtain a compound 1G;
   reacting the compound 1G with benzoic anhydride in the presence of glacial acetic acid to obtain the compound 1H; 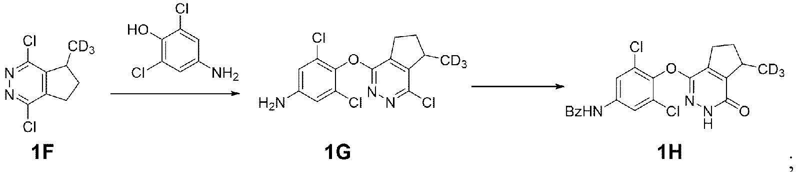
   wherein the organic solvent B is selected from dimethylsulfoxide, acetonitrile, N,N-dimethylformamide, N,N-dimethylacetamide, hexamethylphosphoramide and N-methylpyrrolidone; preferably N,N-dimethylacetamide;
(3) mixing the compound 1H obtained in step (2) with an alkali solution C and an organic solvent C, and reacting same under reflux to obtain a compound 1K; 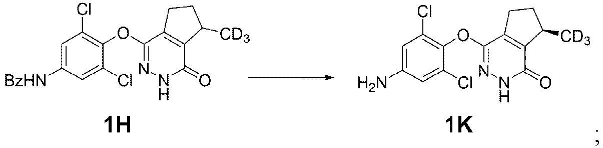 wherein the alkali solution C is selected from a potassium hydroxide solution, a sodium hydroxide solution, a lithium hydroxide solution, a potassium tert-butoxide solution, a lithium tert-butoxide solution, a sodium carbonate solution, a potassium carbonate solution and a cesium carbonate solution; preferably potassium hydroxide and sodium hydroxide; and the organic solvent C is anhydrous methanol, anhydrous ethanol, n-propanol, isopropanol, tert-butanol, n-butanol, toluene, acetone, methyl isobutyl ketone, tetrahydrofuran, 2-methyltetrahydrofuran, dimethylsulfoxide, N,N-dimethylacetamide, hexamethylphosphoramide and N-methylpyrrolidone; preferably anhydrous ethanol and anhydrous methanol;
(4) dissolving a compound 1K obtained in step (3) in water and an organic solvent D, adding an acid B and stirring same, then adding an alkali solution A and an alkali solution B successively, and finally adding N-cyanoacetylurethane for reaction to obtain a compound 1L; 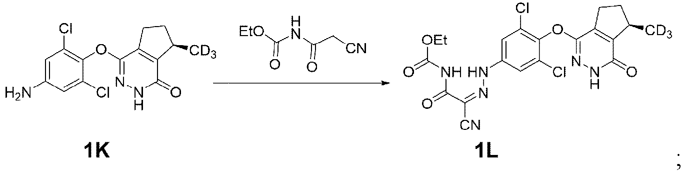 wherein the organic solvent D is selected from formic acid, acetic acid, oxalic acid and propionic acid; preferably acetic acid; the acid B is selected from hydrochloric acid, nitric acid, sulfuric acid, phosphoric acid and perchloric acid; preferably hydrochloric acid; the alkali solution A is a nitrite selected from sodium nitrite, potassium nitrite and ammonium nitrite; and preferably sodium nitrite; and the alkali solution B is an acetate selected from sodium acetate, potassium acetate and ammonium acetate; preferably sodium acetate;
(5) reacting the compound 1L in the presence of an organic solvent B and an alkali solution B to obtain the compound I; 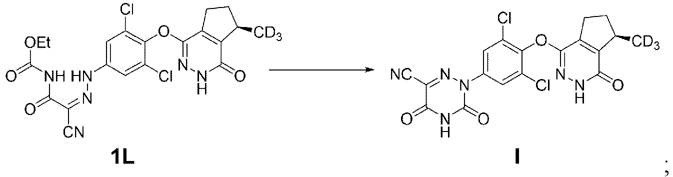 wherein the organic solvent B is selected from dimethylsulfoxide, acetonitrile, N,N-dimethylformamide, N,N-dimethylacetamide, hexamethylphosphoramide and N-methylpyrrolidone; preferably N,N-dimethylacetamide; the alkali solution B is an acetate selected from sodium acetate, potassium acetate and ammonium acetate; preferably sodium acetate.

The present invention has the following beneficial effects:
the method of the present invention has the advantages of easily available raw materials, simple steps, that is, the product is purified by crystallization instead of column chromatography on silica gel or other preparative chromatography methods throughout the entire synthesis process, low costs, good intermediate stability, high purity and high yield, and is suitable for large-scale industrial production.

Unless stated to the contrary, the terms used in the description and claims have the following meanings.

If the compound of the general formula of the present invention has a chiral center, the compound of the general formula can be a racemate or an optical isomer, unless it is clearly marked.

In the present invention, the inert gas refers to a gas that does not participate in the reaction, such as nitrogen.

The carbon, hydrogen, oxygen, sulfur, nitrogen or halogen elements involved in the groups and compounds of the present invention all comprise their isotope forms, and the carbon, hydrogen, oxygen, sulfur, or nitrogen elements involved in the groups and compounds of the present invention are optionally further substituted with 1 to 5 of their corresponding isotopes, wherein the isotopes of carbon comprise ¹²C, ¹³C and ¹⁴C, the isotopes of hydrogen comprise protium (H), deuterium (D, also known as heavy hydrogen), and tritium (T, also known as superheavy hydrogen), the isotopes of oxygen comprise ¹⁶O, ¹⁷O and ¹⁸O, the isotopes of sulfur comprise ³²S, ³³S, ³⁴S and ³⁶S, the isotopes of nitrogen comprise ¹⁴N and ¹⁵N, the isotopes of fluorine comprise ¹⁹F, the isotopes of chlorine comprise ³⁵Cl and ³⁷Cl, and the isotopes of bromine comprise ⁷⁹Br and ⁸¹Br.

In terms of chemistry, an acid refers to a compound in which all cations generated by ionization in an aqueous solution are hydrogen ions, and it can be classified into inorganic acids and organic acids. The acid selected by the present invention is an organic acid or an inorganic acid, wherein the organic acid is, for example, trifluoroacetic acid, pentafluoroacetic acid and heptafluoropropionic acid; and the inorganic acid is, for example, hydrochloric acid and nitric acid.

In theory of acid-base ionization, a base refers to a substance in which all anions generated by ionization in an aqueous solution are OH⁻. The base selected by the present invention is, for example, sodium nitrite, potassium nitrite, sodium acetate, potassium acetate, potassium hydroxide, sodium hydroxide, sodium methoxide, potassium ethoxide, potassium tert-butoxide, sodium tert-butoxide, lithium tert-butoxide, K₃PO₄, K₂CO₃, KHCO₃, Cs₂CO₃, Na₂CO₃, NaHCO₃, KF, pyridine, triethylamine, N,N-diisopropylethylamine and DBU.

### Detailed Description of Embodiments

The technical solutions of the present invention will be described in detail below in conjunction with examples, but the protection scope of the present invention includes but is not limited thereto.

The structure of the compound is determined by nuclear magnetic resonance (NMR) or (and) mass spectrometry (MS). The NMR shift (δ) is given in the unit of 10⁻⁶ (ppm). NMR is determined with (Bruker Avance III 400 and Bruker Avance 300) nuclear magnetic resonance instrument; the solvents for determination are deuterated dimethyl sulfoxide (DMSO-d₆), deuterated chloroform (CDCl₃) and deuterated methanol (CD₃OD); and the internal standard is tetramethylsilane (TMS).
MS is determined with Agilent 6120B (ESI) and Agilent 6120B (APCI);
HPLC is measured with Agilent 1260DAD high pressure liquid chromatography (Zorbax SB-C18 100 × 4.6 mm, 3.5 µM);
Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm.

The known starting materials of the present invention can be synthesized by or according to methods known in the art, or can be purchased from Titan Technology Co., Ltd., Energy Chemical Co., Ltd., Shanghai Demo Co., Ltd., Chengdu Kelong Chemical Co., Ltd., Accela ChemBio Co., Ltd., J&K Scientific Co., Ltd. and other companies.

### Example 1

### (R)-2-(3,5-dichloro-4-((7-(methyl-d3)-1-oxo-2,5,6,7-tetrahydro-1H-cyclopenta[d] pyridazin-4-yl)oxy)phenyl)-3,5-dioxo-2,3,4,5-tetrahydro-1,2,4-triazine-6-carbonitrile

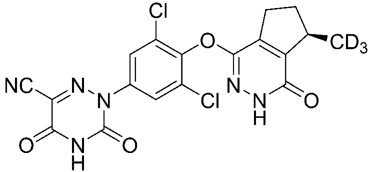

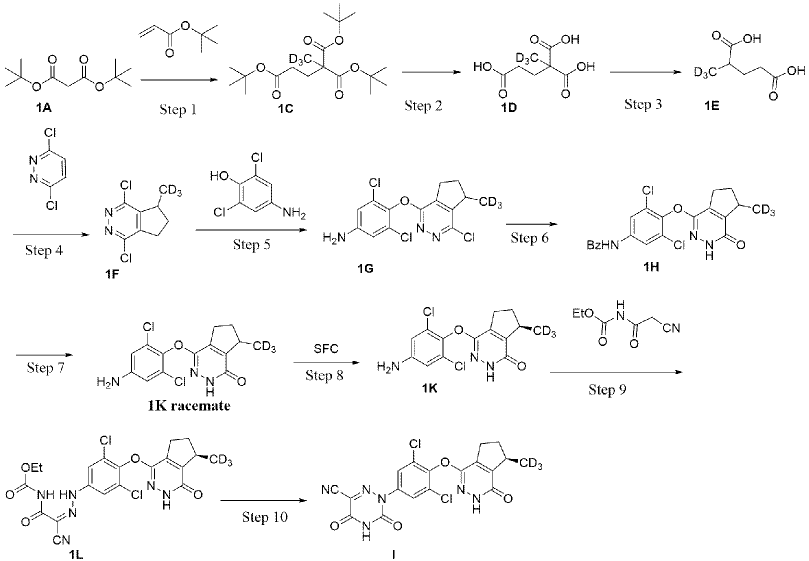

### Step 1: tri-tert-butyl butane-1,3,3-tricarboxylate-4,4,4-d3 (1C)

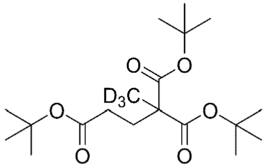

### Method I:

To a 1000 L reaction kettle, 720 L of tetrahydrofuran, 90.0 kg of 1A, 2.33 kg of potassium tert-butoxide and 1.41 kg of tetrabutylammonium bisulfate were added. After the complete addition, the temperature was reduced by 0-10°C under nitrogen protection, and the temperature was controlled below 10°C, 53.3 kg of tert-butyl acrylate was added dropwise. After the complete addition, the mixture was reacted for 1 hour with the temperature maintained constant, and the TLC monitoring (n-hexane/ethyl acetate (v/v) = 10 : 1) showed that the raw materials were basically converted completely. 49.0 kg of potassium tert-butoxide was added to the reaction solution, the temperature was controlled at 0-10°C, and 62.0 kg of deuterated iodomethane was added dropwise to the reaction solution. After the complete addition, the resulting mixture was reacted for another 1 hour, and the TLC monitoring (n-hexane/ethyl acetate (v/v) = 10 : 1) showed that the raw materials were basically converted completely. 115 kg of a 10% ammonium chloride solution was added to the reaction solution, and the aqueous phase was extracted with ethyl acetate (150 L × 2). The organic phases were combined, washed once with 200 L of a saturated aqueous sodium chloride solution and phase-separated. The organic phase was concentrated under reduced pressure at 50 ± 5°C until no fraction appears, so as to obtain 143.0 kg of a liquid as a pale yellow oil. Yield: 95.1%.

¹H NMR (400 MHz, DMSO) δ 2.14-2.10 (m 2H), 1.92-1.88 (m, 2H), 1.39 (s, 27H).

### Method II:

To a 50 L reaction kettle, 16 L of tetrahydrofuran, 2000 g of 1A, 102 g of potassium tert-butoxide and 32 g of Bu₄NHSO₄ were added. After the complete addition, the temperature was reduced by 0-10°C under nitrogen protection, and the temperature was controlled below 10°C, 1185 g of tert-butyl acrylate was added dropwise for about 1-2 hours. After the complete addition, the mixture was reacted for 1 hour with the temperature maintained constant, and the TLC monitoring (n-hexane/ethyl acetate (v/v) = 12 : 1) showed that the raw materials were basically converted completely. 1090 g of potassium tert-butoxide was added to the reaction solution, the temperature was controlled at 0-10°C, and 1407 g of deuterated iodomethane was added dropwise to the reaction solution for about 1 hour. After the complete addition, the mixture was reacted for another 1 hour, and the TLC monitoring (n-hexane/ethyl acetate (v/v) = 12 : 1) showed that the raw materials were basically converted completely. 10 L of a 10% ammonium chloride solution was added to the reaction solution, the aqueous phase was extracted with ethyl acetate (8 L × 2) and the organic phases were combined, washed once with 10 L of a saturated aqueous sodium chloride solution and phase-separated. The organic phase was dried over 1.5 kg of anhydrous sodium sulfate, filtered to remove sodium sulfate and concentrated under reduced pressure at 50 ± 5°C until no fraction appears, so as to obtain 3.756 kg of a liquid as a pale yellow oil. The crude was overweight in 100% yield.

### Step 2: butane-1,3,3-tricarboxylic-4,4,4-d3 acid (1D)

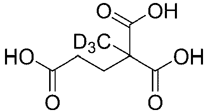

### Method I:

To a 1000 L reaction kettle, 200 L of dichloromethane, 143.0 kg of 1C and 160 L of trifluoroacetic acid were added. After the complete addition, the temperature was raised by 40-45°C and the mixture was refluxed and reacted for about 12 hours, and the TLC monitoring (n-hexane/ethyl acetate (v/v) = 10 : 1) showed that the raw materials were converted completely. After the reaction solution was concentrated under reduced pressure at 50 ± 5°C until no significant fraction appears, 100 L of n-hexane was added, pulped for 2.0 hours, filtered and washed to collect a filter cake. The filter cake was dried at 50 ± 5°C for about 16 hours. The collected material was 65.5 kg of an intermediate 1D as a white solid with a yield of about 85.7% (purity: 95%).

¹H NMR (400 MHz, DMSO) δ 12.66-12.00(brs, 3H), 2.20-2.15 (m, 2H), 1.97-1.92 (m, 2H).
LCMS m/z = 192.1 [M-H]⁺

### Method II:

To a 50 L glass reaction kettle, 8 L of dichloromethane, 3756 g of 1C and 6 L of trifluoroacetic acid were added. After the complete addition, the temperature was raised by 35-40°C and the mixture was refluxed and reacted for about 16 hours, and the TLC monitoring (n-hexane/ethyl acetate (v/v) = 12 : 1) showed that the raw materials were converted completely. After the reaction solution was concentrated under reduced pressure at 50 ± 5°C until no significant fraction appears, 2 L of n-hexane was added, pulped for 0.5 hours, filtered and the filter cake was washed with n-hexane and collected. The filter cake was dried at 50 ± 5°C and a vacuum degree of ≤ -0.07 MPa for about 16 hours. The collected material was 1280 g of an intermediate 1D as a white solid with a two-step yield of about 72% (purity: 95%).

¹H NMR (400 MHz, DMSO) δ 12.66-12.00(brs, 3H), 2.20-2.15 (m, 2H), 1.97-1.92 (m, 2H).
LCMS m/z = 192.1 [M-H]⁺

### Step 3: 2-(methyl-d3)pentanedioic acid (1E)

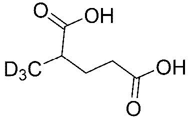

To a 5L reaction bottle, 1280 g of 1D was added, stirring was turned on and the temperature was raised to 130-140°C for reaction for about 5 hours. The reaction solution was cooled to 30-40°C, 1.5 L of dichloromethane was added and stirred for about 10 minutes, and then 1.5 L of n-hexane was added, crystallized with stirring at room temperature for about 16 hours, and then filtered. The filter cake was washed with n-hexane and collected. The filter cake was dried at 50 ± 5°C and a vacuum degree of ≤ -0.07 MPa for about 16 hours. The collected material was 900 g of an intermediate 1E as a white solid with a yield of about 91% (purity: 97%).

¹H NMR (400 MHz, DMSO) δ 12.09(brs, 2H), 2.32-2.23 (m, 1H), 2.21-2.19 (m, 2H), 1.78-1.74(m,1H), 1.58-1.52 (m, 1H).
LCMS m/z =148.1 [M-H]⁺

### Step 4: 1,4-dichloro-5-(methyl-d3)-6,7-dihydro-5H-cyclopenta[d]pyridazine (1F)

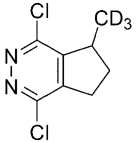

To a 100 L reaction kettle, 22 L of purified water was added, stirring was turned on, 1998 g of 3,6-dichloropyridazine and 2000 g of 1E were added, and heating was turned on to raise the temperature to 35°C. When the internal temperature was raised to 35 ± 5°C, 1146 g of silver nitrate was added. Heating was turned on to raise the internal temperature to 60°C, 306 g of trifluoroacetic acid was added, the dropwise addition of an aqueous (NH₄)₂S₂O₈ solution (10.70 kg of (NH₄)₂S₂O₈ dissolved in 26 L of purified water) was started and reacted to release gases accompanied by an exotherm, with the internal temperature controlled at 75 ± 5°C. After the dropwise addition was complete, the internal temperature was maintained at 75 ± 5°C, and the reaction was continued for half an hour, sampled and monitored by TLC (EA : PE = 1 : 4) until 3,6-dichloropyridazine disappeared.

The temperature was reduced to 40°C, 30 L of methyl tert-butyl ether was added for extraction, the solution was phase-separated and the aqueous phase was extracted once more with 15 L of methyl tert-butyl ether. The organic phases were combined, the aqueous phase was added with 12.5 L of aqueous ammonia and then extracted once with 10 L of methyl tert-butyl ether. The organic phases were combined and washed once with 15 L of a saturated sodium chloride solution, dried over 4 kg of anhydrous sodium sulfate, filtrated and concentrated to obtain a crude.

The crude was mixed with 2 L of dichloromethane and 2 L of petroleum ether for later use. The column was filled with 6 kg of silica gel (200-300 mesh) and loaded after elution with petroleum ether. First, 30-40 L of petroleum ether was used for elution, and then EA : PE = 1 : 10-1 : 20 was used for elution, and the product points were collected. After concentrating most of the eluted phase, a large amount of solid was precipitated. After concentrating to about 3 L of the remaining solvent, same was reduced to 10°C, pulped for 1 h and filtered to obtain the target product 1F. The filtrate could be appropriately recovered, and the product was dried under vacuum at 35 ± 5°C for about 18 h to obtain 0.842 kg of a product as a white solid with a yield of about 40% (purity: 98.0%).

¹H NMR (400 MHz, DMSO) *δ* 3.48-3.46 (m, 1H), 3.20-3.13 (m, 1H), 3.01-2.94 (m, 1H), 2.38-2.33 (m, 1H), 1.84-1.78 (m, 1H).
LCMS m/z = 206.1 [M+1]⁺

### Step 5: 3,5-dichloro-4-((4-chloro-5-(methyl-d3)-6,7-dihydro-5H-cyclopenta[d]pyridazin-1-yl)oxy)aniline (1G)

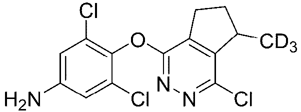

To a 100 L glass reaction kettle, 22 L of N,N-dimethylacetamide was added and stirring was turned on. 2736 g of 1F, 2600 g of 4-amino-2,6-dichlorophenol were added and dissolved completely, then 6488 g of Cs₂CO₃ was added and the mixture, the atmosphere was replaced with nitrogen three times, heating was turned on and the reaction was heated to 90-95°C and stirred for about 20 hours. Samples were collected and delivered for detection and reaction was continued until the content of 1F was < 3.0%.

The reaction solution was cooled to 30°C with stirring, and 20 L of toluene was added. The temperature was continuously reduced to 20°C, 35 L of a 5% sodium chloride solution was added, stirred, and allowed to stand and phase-separated, and the aqueous phase was extracted with toluene (10 L × 2). The organic phases were combined and washed with a 5% sodium chloride solution (20 L × 2), dried over 3.0 kg of anhydrous sodium sulfate, filtered, and concentrated under reduced pressure to obtain a crude 1G as an oil, which was overweight in 100% yield and directly used in the next step.

¹H NMR (400 MHz, DMSO) *δ* 6.71-6.70(d, 2H), 5.66(s, 2H),3.45-3.43 (m, 1H), 3.16-2.97 (m, 2H), 2.45-2.40 (m, 1H), 1.87-1.83 (m, 1H).
LCMS m/z =347.0 [M+1]⁺

### Step 6: N-(3,5-dichloro-4-((7-(methyl-d3)-1-oxo-2,5,6,7-tetrahydro-1H-cyclopenta[d]pyridazin-4-yl) oxy)phenyl)benzamide (1H)

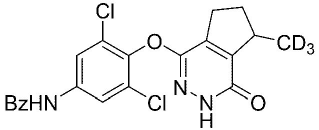

4.62 kg of the crude 1G from the previous step was completely dissolved in 25.0 L of glacial acetic acid and added to a 100 L reaction kettle, 3300 g of benzoic anhydride was added, and the mixture was reacted at room temperature for about 4 hours with stirring turned on. The reaction was monitored by TLC (n-hexane/ethyl acetate = 5/1) until 1G disappeared, then 2722 g of anhydrous sodium acetate was added and the temperature was increased to 120°C for a reaction with stirring for about 18 hours.

The reaction solution was cooled to 60-65°C, and concentrated under reduced pressure to remove most of the acetic acid. After the concentration was completed, 10 L of anhydrous ethanol was added to the residue and uniformly mixed. Then the mixed solution was slowly added to 250 L of water, while maintaining the rapid stirring, and a large amount of solid precipitated during the addition, and stirring was continued for about 0.5 hours after the addition, followed by centrifugation. The filter cake was washed with purified water (20 L × 2) to obtain a crude 1H, which was overweight in 100% yield and directly used in the next step.

¹H NMR (400 MHz, DMSO) *δ* 12.07(s, 1H), 10.55(s, 1H), 8.04(s, 2H), 7.98-7.95(m, 2H), 7.63-7.50(m, 3H), 3.29-3.25 (m, 1H), 3.02-2.90 (m, 2H), 2.39-2.36 (m, 1H), 1.75-1.72 (m, 1H).
LCMS m/z = 433.1 [M+1]⁺

### Step 7: 4-(4-amino-2,6-dichlorophenoxy)-7-(methyl-d3)-2,5,6,7-tetrahydro-1H-cyclopenta[d]pyridazin-1-one (1K racemate)

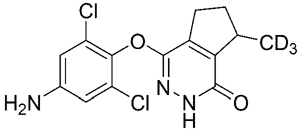

### Method I:

To a 100 L reaction kettle, 5.76 kg of the crude 1H from the previous step, a potassium hydroxide solution (2606 g KOH dissolved in 19.5 L of purified water) and 6.0 L of anhydrous ethanol were added with stirring. After the complete addition, the mixture was heated to reflux and reacted for about 16 hours at 80 ± 5°C, and the raw material was controlled for a complete reaction.

The temperature was reduced to 25°C, 30 L of water was added, the pH was adjusted to 8-9 with an ammonium chloride solid, and 35.0 L of ethyl acetate was added and stirred. The solution was phase-separated. The aqueous phase was extracted with ethyl acetate (15.0 L × 2). The organic phases were combined and washed with a 5% aqueous sodium chloride solution (25 L × 2), dried over 3.0 Kg of anhydrous sodium sulfate, filtered, and concentrated until no significant fraction flowed out, so as to obtain a crude.

The crude and 7.0 L of a 10% aqueous dioxane solution were heated for complete dissolution, cooled to room temperature, and crystallized with stirring for about 16 hours, followed by filtration to obtain a wet product, which was repeated purified twice and dried under vacuum at 50°C for about 12 hours to obtain 1507 g of a yellow solid, with a total yield of 34.5% (purity: 98.5%) in three steps (steps 5, 6 and 7).

¹H NMR (400 MHz, DMSO) *δ* 11.98 (s, 1H), 6.67 (m, 2H), 5.60 (s, 2H), 3.30-3.19 (m, 1H), 3.03-2.93 (m, 1H), 2.90-2.70 (m, 1H), 2.35 (m,1H), 1.69 (m, 1H).
LCMS m/z = 329.0 [M+1]⁺

### Method II:

To a 100 L reaction kettle, 1.0 kg of the crude 1H from the previous step, a sodium hydroxide solution (1.12 kg of NaOH dissolved in 10 kg of purified water) and 4.46 kg of anhydrous methanol were added with stirring. After the complete addition, the mixture was heated to reflux and reacted for about 12 hours at 80 ± 5°C, and the raw material 1H was controlled for a basically complete reaction.

The temperature was reduced to 25°C, 10 kg of water and 10 kg of ethyl acetate were added and stirred. The solution was phase-separated. The aqueous phase was extracted with 10 kg of ethyl acetate. The organic phases were combined and washed with 10 kg of a 5% aqueous sodium chloride solution, dried over 3.0 kg of anhydrous sodium sulfate, filtered, and concentrated until no significant fraction flowed out, so as to obtain a crude.

The crude and 2.0 L of a 10% aqueous dioxane solution were heated for complete dissolution, cooled to room temperature, and crystallized with stirring for about 6 hours, followed by filtration to obtain a wet product, which was repeated purified twice and dried under vacuum at 50°C for about 12 hours to obtain 228 g of a yellow solid, with a total yield of 35.0% (purity: 98.0%) in three steps (steps 5, 6 and 7).

### Step 8: (R)-4-(4-amino-2,6-dichlorophenoxy)-7-(methyl-d3)-2,5,6,7-tetrahydro-1H-cyclopenta[d]pyridazin-1-one (1K)

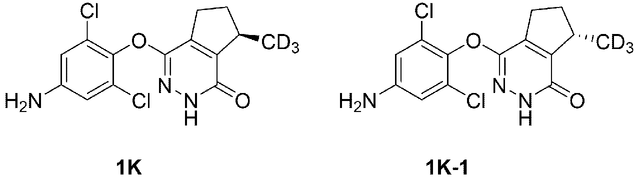

3298 g of the racemate was separated by chiral resolution to obtain two optical isomers:
compound 1K (retention time: 1.583 s, 1230 g, off-white solid, ee% = 99.60%, yield 37.3%); and
compound 1K-1 (retention time: 1.926 s, 1255 g, off-white solid, ee% = 99.76%, yield 38.1%).

### Resolution conditions:

Instrument: MG III preparative SFC; column: Whelk O1(S, S), 300 × 50 mm I.D., 10 µm;
mobile phase: A: CO2, B: methanol; gradient: B 40%; flow rate: 200 mL/min; back pressure: 100 bar;
column temperature: 38°C; wavelength: 220 nm; period: 4.5 min; sample preparation: the racemate was dissolved in methanol/dichloromethane to achieve 50 mg/ml; and injection: 17 ml/injection.

### Compound 1K

¹H NMR (400 MHz, DMSO) δ 11.98 (s, 1H), 6.67 (s, 2H), 5.60 (s, 2H), 3.30-3.19 (m, 1H), 3.03-2.93 (m, 1H), 2.90-2.70 (m, 1H), 2.35 (dtd,1H), 1.69 (ddt, 1H).
LCMS m/z = 329.1 [M+1]⁺

### Compound 1K-1

¹H NMR (400 MHz, DMSO) δ 11.98 (s, 1H), 6.68 (d, 2H), 5.60 (s, 2H), 3.29-3.18 (m, 1H), 2.97 (tdd, 1H), 2.90-2.72 (m, 1H), 2.35 (dtd, 1H), 1.69 (ddt, 1H).
LCMS m/z = 329.0 [M+1]⁺

### Step 9: ethyl (R,Z)-(2-cyano-2-(2-(3,5-dichloro-4-((7-(methyl-d3)-1-oxo-2,5,6,7-tetrahydro-1H-cyclopenta[d]pyridazin-4-yl)oxy)phenyl)hydrazineylidene)acetyl)carbamate (1L)

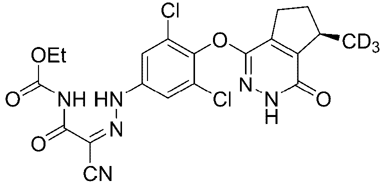

### Method I:

To a 100 L reaction kettle, 16.0 kg of acetic acid, 4.0 kg of purified water and 2.0 kg of 1K were added with stirring. The temperature was reduced to 0 ± 5°C, then 2.36 kg of hydrochloric acid was added. After the complete addition, the temperature was maintained at 0 ± 5°C with stirring for about 20 minutes. A sodium nitrite solution (0.5 kg of sodium nitrite dissolved in 1.0 kg of purified water) was dropwise added with the temperature being controlled at 0 ± 5°C, and after the addition, the temperature was maintained at 0 ± 5°C for reaction for 2 hours. The temperature was controlled at 5 ± 5°C and a sodium acetate solution (1.5 kg of sodium acetate dissolved in 6.0 kg of purified water) was added dropwise, then 0.99 kg of N-cyanoacetourethane was added. After the complete addition, the temperature was increased to 10 ± 5°C for a reaction for about 2 hours. Then a sample was taken for HPLC monitoring, after which time samples were taken at each about 2-hour interval, and the reaction was not stopped until the content of 1K was determined by HPLC to be ≤ 1.0%.

After the completion of the reaction, the temperature was controlled to 10 ± 5°C, and 30.0 kg of purified water was added to the reaction kettle. After the complete addition, the temperature was controlled at 10 ± 5°C with stirring continued for 1 hour. Filtration was carried out, and the filter cake was washed with 3.0 kg of purified water. The filter cake and 12.6 kg of anhydrous ethanol were added to a 100 L reaction kettle, heated to 50 ± 5°C, and stirred for about 1 hour. The mixture was cooled to 20 ± 5°C, stirred for 0.5 hours and filtered, and the filter cake was washed once with 1.26 kg of anhydrous ethanol.

The filter cake was dried at 55 ± 5°C and a vacuum degree of ≤ -0.07 MPa for about 17 hours, and an intermediate 1L was obtained and collected, weighing 2.6327 kg. The yield was based on 1K and was 87.3% (purity: 98.18%).

¹H NMR (400 MHz, DMSO) δ 12.08 (d, 2H), 10.88 (s, 1H), 7.99 (s, 2H), 4.21 (q, 2H), 3.30-3.17 (m, 1H), 3.08-2.95 (m, 1H), 2.95-2.80 (m, 1H), 2.38 (ddd, 1H), 1.78-1.63 (m, 1H), 1.28 (t, 3H).
LCMS m/z = 496.1 [M+1]⁺

### Method II:

To a 100 L reaction kettle, 10.0 kg of acetic acid, 2.4 kg of purified water and 1.20 kg of 1K were added with stirring. The temperature was reduced to 0 ± 5°C, then 1.42 kg of hydrochloric acid was added. After the complete addition, the temperature was maintained at 0 ± 5°C with stirring for about 30 minutes. A sodium nitrite solution (0.334 kg of sodium nitrite dissolved in 0.6 kg of purified water) was dropwise added with the temperature being controlled at 0 ± 5°C, and after the addition, the temperature was maintained at 0 ± 5°C for reaction for 2 hours. The temperature was controlled at 5 ± 5°C and a sodium acetate solution (1100 kg of sodium acetate dissolved in 3.6 kg of purified water) was added dropwise, then 0.683 kg of N-cyanoacetourethane was added. After the complete addition, the temperature was increased to 10 ± 5°C for a reaction for about 2 hours. Then a sample was taken for HPLC monitoring, and the reaction was not stopped until the content of 1K was determined by HPLC to be ≤ 1.0%.

After the completion of the reaction, the temperature was controlled to 10 ± 5°C, and 18.0 kg of purified water was added to the reaction kettle. After the complete addition, the temperature was controlled at 10 ± 5°C with stirring continued for 1 hour. Filtration was carried out, and the filter cake was washed with 4.0 kg of ethyl acetate.

The filter cake was dried at 55 ± 5°C and a vacuum degree of ≤ -0.07 MPa for about 16 hours, and an intermediate 1L was obtained and collected, weighing 1.845 kg. It was overweight in 100% yield (purity: 98.64%).

### Step 10: (R)-2-(3,5-dichloro-4-((7-(methyl-d3)-1-oxo-2,5,6,7-tetrahydro-1H-cyclopenta[d]pyridazin-4-yl)oxy)phenyl)-3,5-dioxo-2,3,4,5-tetrahydro-1,2,4-triazine-6-carbonitrile (I)

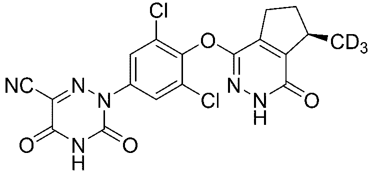

### Method I:

To a 100 L reaction kettle, 12.40 kg of N,N-dimethylacetamide, 2.6269 kg of 1L and 0.54 kg of sodium acetate were added with stirring. After the complete addition, the temperature was increased and the internal temperature was maintained at 115 ± 5°C for a reaction for about 2 hours. Then a sample was taken for HPLC monitoring, after which time samples were taken at each about 2-hour interval, and the reaction was not stopped until the content of 1L was determined by HPLC to be ≤ 1.0%.

After the completion of the reaction, the temperature was reduced to 60 ± 5°C, 0.788 kg of purified water was added to the reaction solution. After the addition, the reaction solution was filtered while still hot and washed with 1.24 kg of N,N-dimethylacetamide, and the filtrate was transferred to a 100 L reaction kettle. The temperature was controlled at 60 ± 5°C, and 0.39 kg of acetic acid and 13.66 kg of purified water were added. After the completion of the reaction, the temperature was reduced to 10 ± 5°C and crystallized with stirring for about 2 hours. Filtration was carried out, and the filter cake was washed with 2.63 kg of purified water and 2.11 kg of anhydrous ethanol, and dried with suction to collect the filter cake, so as to obtain a crude I (in 100% yield). The crude was directly used in the next step for reaction.

To a 100 L reaction kettle, 12.35 kg of anhydrous ethanol and the total amount of the crude I from the previous step were added with stirring, heated to 75 ± 5°C, and reacted with stirring for about 4 hours. The mixture was cooled to 5 ± 5°C, crystallized with stirring for about 12 hours and filtered, and the filter cake was washed with ethanol twice, with the amount of 1.05 kg each time, so as to obtain a refined wet product I.

The refined wet product was dried at 55 ± 5°C and a vacuum degree of ≤ - 0.07 MPa for about 17 hours to obtain a finished product I. The yield was based on 1L, the weight was 2.02555 kg, and the yield was 85.0% (purity: 99.74%).

¹H NMR (400 MHz, DMSO) δ 13.26 (s, 1H), 12.09 (s, 1H), 7.79 (s, 2H), 3.32-3.24 (m, 1H), 3.10-2.99 (m, 1H), 2.96-2.88 (m, 1H), 2.45-2.31 (m, 1H), 1.77-1.69 (m, 1H).
LCMS m/z = 450.0 [M+1]⁺.

### Method II:

To a 100 L reaction kettle, 8.53 kg of N,N-dimethylacetamide, 1.831 kg of 1L and 0.434 kg of sodium acetate were added with stirring. After the complete addition, the temperature was increased and the internal temperature was maintained at 120 ± 5°C for a reaction for about 2 hours. Then a sample was taken for HPLC monitoring, and the reaction was not stopped until the content of 1L was determined by HPLC to be ≤ 1.0%.

After the completion of the reaction, the temperature was reduced to 20 ± 5°C, 0.52 kg of acetic acid was added, and the reaction solution was added to 50.0 kg of ice water and a large number of solids were precipitated. Filtration was carried out, and the filter cake was washed with purified water and 2.11 kg of anhydrous ethanol, and dried with suction to collect the filter cake, so as to obtain a crude I (in 100% yield). The crude was directly used in the next step for reaction.

To a 100 L reaction kettle, 10.0 kg of anhydrous isopropanol and the total amount of the crude I from the previous step were added with stirring, heated to 80 ± 5°C, and reacted with stirring for about 3 hours. The mixture was cooled to 5 ± 5°C, crystallized with stirring for about 12 hours and filtered, and the filter cake was washed with isopropanol to obtain a refined wet product I.

The refined wet product was dried at 55 ± 5°C and a vacuum degree of ≤ - 0.07 MPa for about 12 hours to obtain a finished product I. The yield was based on 1L, the weight was 1.582 kg, and the yield was 82.6% (purity: 99.23%).

## Claims

1. Compounds of formulae (1E) and (1H), or a pharmaceutically acceptable salt thereof,

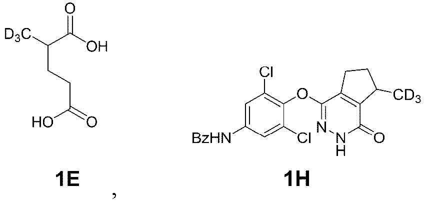

2. A preparation method of a compound of formula (1F), **characterized by** comprising the steps of 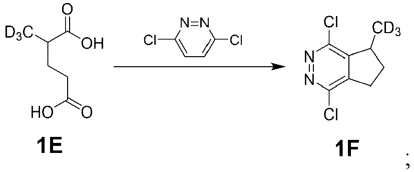 after the reaction of a compound 1E with 3,6-dichloropyridazine, water and silver nitrate, adding an acid A and an oxidant for reaction to obtain the compound 1F.

3. A preparation method of a compound of formula (1E) according to claim 1 or 2, **characterized by** comprising the steps of 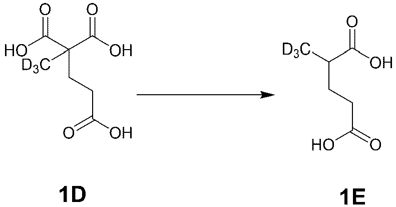 reacting a compound 1D with stirring at 100-180°C to obtain the compound 1E.

4. The preparation method according to claim 3, **characterized by** further comprising the steps of 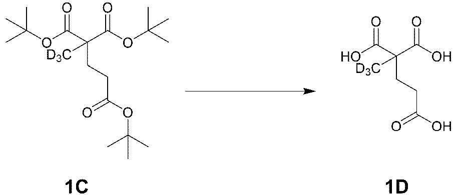 reacting a compound 1C with an organic solvent F and an acid A under reflux to obtain the compound 1D.

5. The preparation method according to claim 4, **characterized in that** the organic solvent F is selected from dichloromethane, methyl acetate, dimethyl carbonate, propylene glycol methyl ether acetate, dimethyl nylon acid, acetonitrile, toluene, ethyl acetate, isopropyl acetate, acetone, 2-methyltetrahydrofuran, tetrahydrofuran and 1,4-dioxane; and the acid A is selected from trifluoroacetic acid, pentafluoroacetic acid, heptafluoropropionic acid, hydrochloric acid, sulfuric acid, phosphoric acid, formic acid, benzenesulfonic acid, methanesulfonic acid and p-toluenesulfonic acid.

6. The preparation method according to claim 4, **characterized by** further comprising the steps of 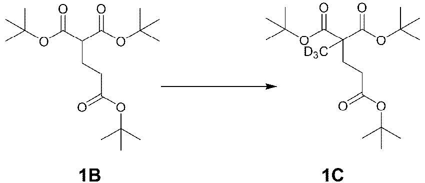 reacting a compound 1B with a deuterated reagent in the presence of a base to obtain the compound 1C.

7. The preparation method according to claim 6, **characterized in that** the deuterated reagent is selected from deuterated iodomethane, deuterated bromomethane, deuterated chloromethane, D₃-p-toluenesulfonyl methyl ester and D₃-methylsulfonyl methyl ester.

8. The preparation method according to claim 6, **characterized by** further comprising the steps of 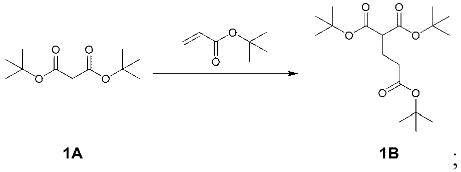 adding a compound 1A, a base and a catalyst to an organic solvent E, followed by adding t-butyl acrylate under the protection of an inert gas to obtain the compound 1B.

9. The preparation method according to claim 6 or 8, **characterized in that** the base is selected from sodium methoxide, potassium ethoxide, potassium tert-butoxide, sodium tert-butoxide, lithium tert-butoxide, sodium acetate, potassium acetate, K₃PO₄, K₂CO₃, KHCO₃, Cs₂CO₃, Na₂CO₃, NaHCO₃, KF, pyridine, triethylamine, N,N-diisopropylethylamine and DBU; the catalyst is tetrabutylammonium bisulfate; and the organic solvent E is selected from tetrahydrofuran, N,N-dimethylacetamide, dimethylformamide, acetonitrile, acetone, methyl isobutyl ketone, toluene, pyridine, dichloromethane, ethyl acetate, 2-methyltetrahydrofuran or 1,4-dioxane.

10. A preparation method of a compound of formula (I), **characterized by** comprising the steps of 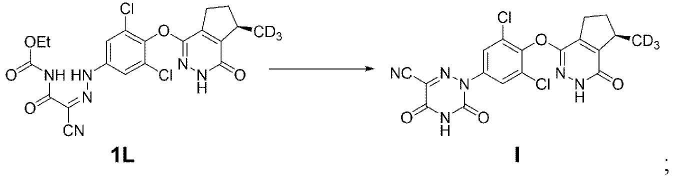 reacting a compound 1L in the presence of an organic solvent B and an alkali solution B to obtain the compound I; the organic solvent B is selected from dimethylsulfoxide, acetonitrile, N,N-dimethylformamide, N,N-dimethylacetamide, hexamethylphosphoramide and N-methylpyrrolidone.

11. The preparation method according to claim 10, **characterized by** further comprising the steps of 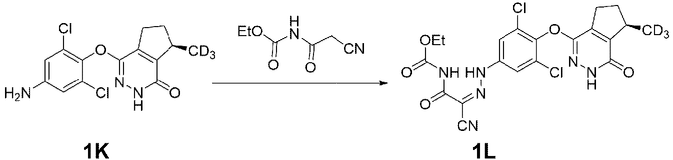 dissolving a compound 1K in water and an organic solvent D, adding an acid B and stirring same, then adding an alkali solution A and an alkali solution B, and N-cyanoacetylurethane successively for reaction to obtain the compound 1L.

12. The preparation method according to claim 10 or 11, **characterized in that** the organic solvent D is selected from formic acid, acetic acid, oxalic acid and propionic acid; the acid B is selected from hydrochloric acid, nitric acid, sulfuric acid, phosphoric acid and perchloric acid; the alkali solution A is a nitrite selected from sodium nitrite, potassium nitrite and ammonium nitrite; and the alkali solution B is an acetate selected from sodium acetate, potassium acetate and ammonium acetate.

13. The preparation method according to claim 11, **characterized by** further comprising the steps of 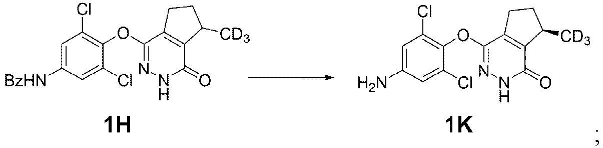 mixing a compound 1H with an alkali solution C and an organic solvent C, and reacting same under reflux to obtain the compound 1K.

14. The preparation method according to claim 13, **characterized in that** the alkali solution C is selected from a potassium hydroxide solution, a sodium hydroxide solution, a lithium hydroxide solution, a potassium tert-butoxide solution, a lithium tert-butoxide solution, a potassium carbonate solution and a cesium carbonate solution; and the organic solvent C is selected from anhydrous methanol, anhydrous ethanol, n-propanol, isopropanol, tert-butanol, n-butanol, toluene, acetone, methyl isobutyl ketone, tetrahydrofuran, 2-methyltetrahydrofuran, dimethylsulfoxide, N,N-dimethylacetamide, hexamethylphosphoramide and N-methylpyrrolidone.

15. The preparation method according to claim 13, **characterized by** further comprising the steps of 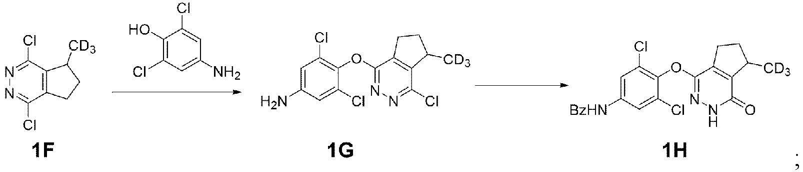
reacting a compound 1F and 4-amino-2,6-dichlorophenol in the presence of an organic solvent B and cesium carbonate under the protection of an inert gas to obtain a compound 1G; and
reacting the compound 1G with benzoic anhydride in the presence of glacial acetic acid to obtain the compound 1H.

16. The preparation method according to claim 15, **characterized in that** the organic solvent B is selected from dimethylsulfoxide, acetonitrile, N,N-dimethylformamide, N,N-dimethylacetamide, hexamethylphosphoramide and N-methylpyrrolidone.

17. The preparation method according to claim 15, **characterized by** further comprising the steps of 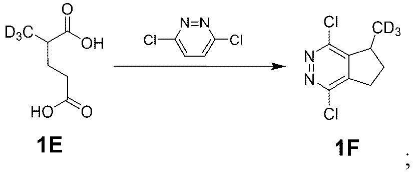 after the reaction of a compound 1E with 3,6-dichloropyridazine, water and silver nitrate, adding an acid A and an oxidant for reaction to obtain the compound 1F.

18. The preparation method according to claim 2 or 17, **characterized in that** the acid A is selected from trifluoroacetic acid, pentafluoroacetic acid, heptafluoropropionic acid, sulfuric acid, hydrochloric acid, phosphoric acid, nitric acid, formic acid, acetic acid, benzenesulfonic acid, methanesulfonic acid and p-toluenesulfonic acid; and the oxidant is selected from ammonium persulfate, potassium persulfate, sodium persulfate, t-butylhydroperoxide and hydrogen peroxide.
